# EUROPEAN PATENT SPECIFICATION

(11) **EP 2 955 376 B1**
(45) Date of publication and mention of the grant of the patent: **16.12.2020**
(21) Application number: 14748966.0
(22) Date of filing: 06.02.2014
(51) Int. Cl.: F04B 43/12, F04B 43/08

(54) **TUBING PUMP**
SCHLAUCHPUMPE
POMPE À TUBES

(30) Priority: 06.02.2013 US 201313760794
(43) Date of publication of application: 16.12.2015
(73) Proprietor: Adamant Namiki Precision Jewel Co., Ltd., Tokyo 123-8511 (JP); Namiki Precision Singapore Pte. Ltd., Singapore 068804 (SG)
(72) Inventor: KOYAMA, Masahiro, 068804 (SG); KASHIWAGI, Makoto, Tokyo 123-8511 (JP)
(74) Representative: Prinz & Partner mbB
(86) International application number: PCT/JP2014/052725
(87) International publication number: WO 2014/123178

(56) References cited:
- EP-A1- 0 426 273
- EP-A1- 2 568 178
- WO-A2-2011/082135
- GB-A- 2 006 347
- JP-A- S5 874 881
- JP-A- 2013 060 813
- JP-U- H0 347 481
- US-A- 2 412 397
- US-A- 3 606 596
- US-A- 3 998 103
- US-A- 4 936 760
- US-A- 5 151 019
- US-A1- 2003 017 066
- US-A1- 2003 181 865

## Description

### TECHNICAL FIELD

The present invention relates to a tubing pump according to claim 1 that is mainly used as an infusion pump for medical use and that transfers a liquid in a tube, which is detachably installed, using a pump mechanism.

### 1. BACKGROUND ART

In the related art, infusion pumps for medical use include a shuttle-type infusion pump in which a tube is disposed between two V-grooves and is repeatedly pressed by a reciprocation of any one of the two V-grooves.

As an example of the shuttle-type infusion pump, Patent Document 1 discloses a shuttle-type infusion pump in which a tube is disposed between a V-groove constituted by an upper jaw 220 and a lower jaw 222 and a V-groove of a shuttle 200 and is repeatedly pressed by a reciprocation of the shuttle 200. In the infusion pump disclosed in Patent Document 1, an upstream valve 412 is disposed on the upstream side of the shuttle 200, and a downstream valve 414 is disposed on the downstream side thereof. The upstream valve 412 and the downstream valve 414 block up an infusion tube or release the blocking-up state at an appropriate timing in association with the reciprocation of the shuttle 200, and thus a liquid in the infusion tube is transferred. Operation ranges of the shuttle 200, the upstream valve 412, and the downstream valve 414 are defined by one cam 100 in which cam profiles for defining operations of the shuttle 200, the upstream valve 412, and the downstream valve 414 are individually formed.

As another example of the shuttle-type infusion pump, Patent Document 2 discloses a shuttle-type infusion pump in which a tube is disposed between a V-groove of a V-groove-shaped fixed component 22 and a V-groove of a V-groove-shaped driving component 12A and is repeatedly pressed by the reciprocation action of the V-groove-shaped driving component 12A.

The shuttle-type infusion pumps as disclosed in Patent Document 1 and Patent Document 2 realize an excellent flow rate accuracy as compared with, for example, a peristaltic-type infusion pump disclosed in Patent Document 3 in which an infusion tube is pressed by peristalsis of all of a plurality of fingers.

US 3,606,596 shows a tubing pump that transfers a liquid in a detachably installed tube by using a pump mechanism with a valve mechanism unit that blocks up and opens the installed tube and a tube pressing mechanism unit that repeatedly presses the tube, where the tube pressing mechanism unit has a pump block that is pivotally held by a shaft.

GB 2 006 347 A shows a tubing pump with a tube pressing mechanism that includes several adjacent pump blocks that press the tube in a predetermined order to transport liquid through the tube, the package of pump blocks being reciprocatably guided between four guide shafts arranged externally around the pump blocks.

EP 0 426 273 A1 shows a further tubing pump, having a pump block for pressing the tube arranged between two valves for blocking and opening the tube. This document however does not disclose that the pump block is movably held by at least two guide shafts extending into the pump block.

EP 2 568 178 A1, published after the priority date of the present application, shows a tubing pump having a pump block that is reciprocated by a cam drive and is guided in a carrier by two guide shafts on each side of the pump block extending into guiding grooves in the carrier.

Generic JP H03 47481 U2 shows a tubing pump for transferring liquid in detachably installed tube. The pump is used e.g. for pumping concrete and has a pump block with several cam-operated compression bodies acting from both sides on an elastic tube. The compression bodies are guided on guide shafts in a reciprocating direction parallel to the guide shafts.

### CITATION LIST

### PATENT DOCUMENT

(Patent Document 1) Japanese Laid-Open Patent Publication No. hei 11-508017
(Patent Document 2) International Patent Publication No. WO2009/133705
(Patent Document 3) Japanese Laid-Open Patent Publication No. hei 5-277183

### SUMMARY OF THE INVENTION

### PROBLEM TO BE SOLVED BY THE INVENTION

However, further improvement of flow rate accuracy, particularly in infusion pumps for medical use is required.

### MEANS FOR SOLVING PROBLEM

According to an aspect of the present invention, there is provided a tubing pump that transfers a liquid in a tube, with the features of claim 1.

According to the above configuration, since the jouncing of a driving unit may be prevented, a liquid in an infusion tube may be transferred at further stable flow rate accuracy.

The tube pressing mechanism unit is provided with a facing component which does not operate when transferring the liquid in the tube, the facing component being disposed at a position facing the pump block. A first groove portion having an approximately V shape is formed in the pump block. A second groove portion having an approximately V shape is formed in the facing component. The tube is repeatedly pressed between the first groove portion and the second groove portion when the pump block continuously reciprocates.

According to the above configuration, in the process of repeatedly pressing the tube, the deformation state of the tube due to the pressing may be restored before the tube is to be pressed next, and thus the liquid in the infusion tube may be transferred at further stable flow rate accuracy.

According to another aspect of the present invention, the valve mechanism unit is disposed on the upstream side and the downstream side. The tube pressing mechanism unit is disposed between the valve mechanism unit on the upstream side and the valve mechanism unit on the downstream side. An operation range of the pump block disposed in the tube pressing mechanism unit is determined by a pump cam. An operation range of an upstream side valve of the valve mechanism unit on the upstream side is determined by a first valve cam. An operation range of a downstream side valve of the valve mechanism unit on the downstream side is determined by a second valve cam. The pump cam, the first valve cam, and the second valve cam are different cam components to each other.

According to the above configuration, the opening and closing timings of the valve may be further appropriately set, and thus the liquid in the infusion tube may be transferred at further stable flow rate accuracy.

### EFFECT OF THE INVENTION

According to the invention, an effect to be able to constitute the tubing pump which can improve the flow rate accuracy more is provided.

### BRIEF DESCRIPTION OF DRAWINGS

FIG. 1 is a perspective view showing an appearance of a tubing pump according to the present invention;
FIG. 2 is a perspective view showing a state where a front door of the tubing pump according to the present invention is opened;
FIG. 3 is an exploded perspective view showing a structure of the tubing pump according to the present invention;
FIGS. 4A to 4C are diagrams showing a positional relationship between a pump cam and a pump block in the tubing pump according to the present invention;
FIG. 5 is a diagram showing a cam profile of the pump cam in the tubing pump according to the present invention;
FIGS. 6A to 6D are schematic diagrams showing a deformation-restoration state of a tube in the tubing pump according to the present invention;
FIGS. 7A and 7B are diagrams showing a positional relationship between a valve cam and a valve in the tubing pump according to the present invention; and
FIG. 8 is a timing chart showing operation timings of the pump block and the valve in the tubing pump according to the present invention.

### MODE(S) FOR CARRYING OUT THE INVENTION

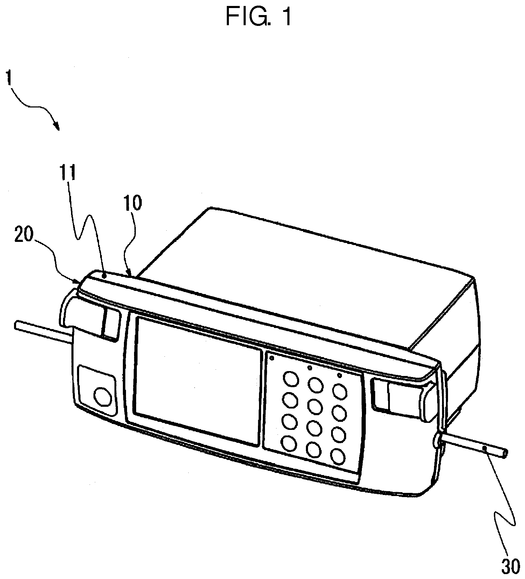

Hereinafter, an embodiment of the present invention will be described in detail with reference to the accompanying drawings. Also, the embodiment corresponds to the drawings of FIGS. 1 to 8. In the following description, reference numerals are used to describe elements when there is any appropriate drawing to refer to.

### EMBODIMENT

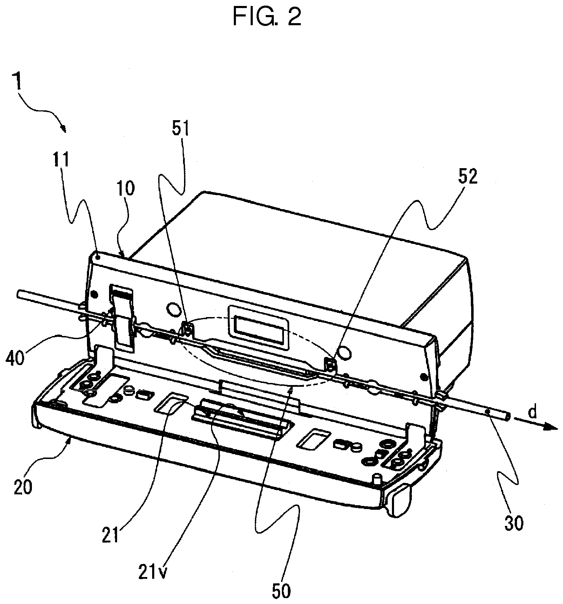

FIGS. 1 and 2 show an appearance of a tubing pump 1 to which a pump mechanism according to the present invention is applied, wherein the tubing pump 1 is an apparatus that compulsorily causes a liquid (for example, a medicinal fluid or a nutritional agent) in a tube to flow by a pump so as to inject the liquid into a patient's body. The tubing pump 1 includes a door 20 that opens and closes a front portion of a pump body 10. FIG. 1 shows a closed state of the door 20, and FIG. 2 shows an opened state of the door 20.

The pump body 10 is formed to have an approximately rectangular box shape. The pump body 10 has an upright base 11 that is opened and closed by the door 20 on a front side thereof. A tube 30 that is clamped by a clamping device 40 is laterally penetrated the base 11 (see FIG. 2). The clamping state of the tube 30 due to the clamping device 40 is released when the door 20 is closed.

The base 11 is a thick part having an approximately plate shape and includes a pump mechanism 50 that compulsorily transfers the liquid in the installed tube 30. The pump mechanism 50 compulsorily transfers the liquid in the tube 30 from the upstream side to the downstream side, i.e., in a direction d, by appropriately combining a pressing-opening operation with respect to the installed tube 30, an opening-closing operation of a valve 51 on the upstream side thereof, an opening-closing operation of a valve 52 on the downstream side thereof, and the like (see FIG. 2).

### <Configuration of Pump Mechanism>

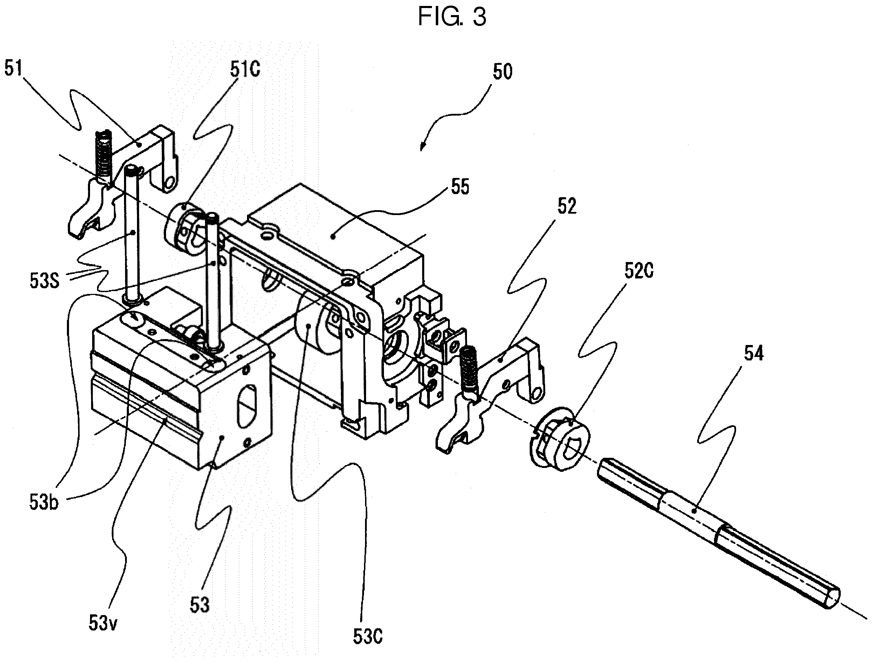

FIG. 3 is an exploded perspective view showing components of the pump mechanism 50. The pump mechanism 50 includes the upstream side valve 51, an upstream side valve cam 51C, the downstream side valve 52, a downstream side valve cam 52C, a pump block 53, a pump block cam 53C, a guide shaft 53S, a shaft 54, a pump block case 55, and the like.

Among these components, a tube pressing mechanism unit is formed by including the pump block 53, the pump block cam 53C, and the guide shaft 53S as main components.

An upstream side valve mechanism unit is formed in the pump mechanism 50 by including the upstream side valve 51 and the upstream side valve cam 51C as main components. A downstream side valve mechanism unit is formed in the pump mechanism 50 by including the downstream side valve 52 and the downstream side valve cam 52C as main components.

The upstream side valve cam 51C, the downstream side valve cam 52C, and the pump block cam 53C are fixed to the shaft 54. The shaft 54 continuously rotates by using an electric motor (not shown) as a power source.

When the shaft 54 continuously rotates, the upstream side valve cam 51C, the downstream side valve cam 52C, and the pump block cam 53C respectively cause the upstream side valve 51, the downstream side valve 52, and the pump block 53 to reciprocate in predetermined ranges that are determined by the respective cam profile.

At this time, the tube pressing mechanism unit is associated with the upstream and downstream side valve mechanism units so as to repeatedly press the tube 30 at an appropriate timing, and to block up the tube 30 or to release the blocking-up of the tube 30 on the upstream side and the downstream side, and thus the liquid in the infusion tube 30 is transferred.

### <Tube Pressing Mechanism Unit>

As described above, the tube pressing mechanism unit includes the pump block 53, the pump block cam 53C, and the guide shaft 53S.

The position of the pump block 53 that vertically reciprocates is determined by the position of the pump block cam 53C that rotates around the shaft 54 as a rotating shaft.

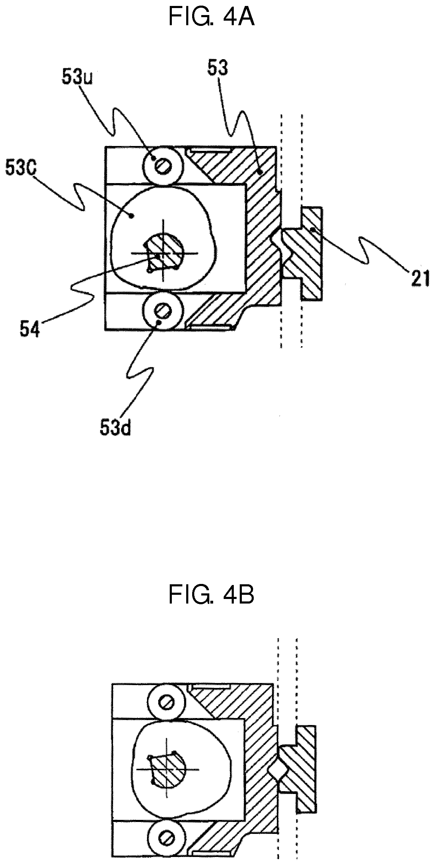

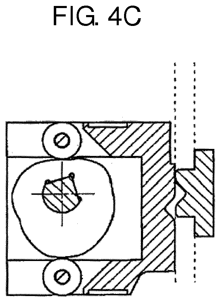

FIGS. 4A to 4C show a cam profile of the pump block cam 53C. The position of the pump block 53 is determined by the position at which a cam surface of the pump block cam 53C having the cam profile shown in FIGS. 4A and 4C contacts an upper bearing unit 53u or a lower bearing unit 53d of the pump block 53 when the pump block 53 vertically moves from a state where the pump block 53 is located at an intermediate position.

FIG. 4A shows a positional relationship between the pump block 53 and the pump block cam 53C in a state where the pump block 53 is located at the uppermost position. FIG. 4B shows a positional relationship between the pump block 53 and the pump block cam 53C in a state where the pump block 53 is located at the intermediate position. FIG. 4C shows a positional relationship between the pump block 53 and the pump block cam 53C in a state where the pump block 53 is located at the lowermost position.

In addition, the pump block 53 is held so as to be movable only vertically by causing the guide shaft 53S to pass through ball bushing units 53b provided on the upstream side and the downstream side.

The pump block 53 is held by the guide shaft 53S, and thus the pump block 53 may be prevented from jouncing in other directions when the pump block 53 vertically reciprocates. In addition, the pump block 53 is prevented from jouncing, thereby allowing vibration and noise generated during the operation of the tubing pump 1 to be suppressed and contributing to the improvement of flow rate accuracy as an infusion pump.

A V-groove 53v, which is a concave portion having an approximately V shape, is formed in the pump block 53, and the tube 30 is disposed along the V-groove 53v.

A pump block facing component 21 is fixed to the door 20 at a position facing the pump block 53 when the door 20 is closed.

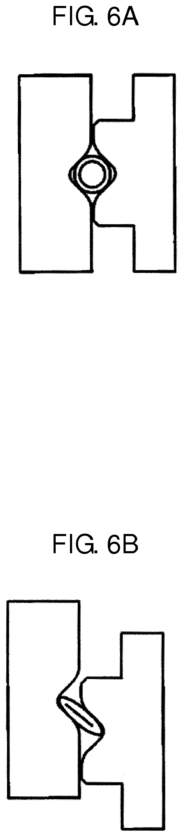

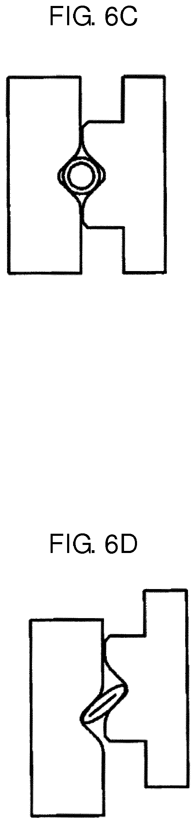

The tube 30 is disposed in a diamond-shaped space formed by causing a V-groove 21v, which is a recess portion having an approximately V shape and is formed in the pump block facing component 21, and the V-groove 53v of the pump block 53 to face each other (see FIGS. 6A and 6C).

From this state, when the pump block 53 vertically reciprocates, the tube 30 is repeatedly pressed (see FIGS. 6B and 6D).

When the diamond-shaped space is formed and is then released from a state (FIG. 6B) where the tube 30 is pressed during the operation of the pump block 53, the shape of the tube 30 tries to return the state before being pressed by elastic force of a material of the tube 30 and pressure of the liquid flowing into the tube 30.

At this time, the infusion tube 30 is pressed from both sides thereof by an inclined surface of the V-groove 53v and an inclined surface of the V-groove 21v in addition to the elastic force of the material of the tube 30 and the pressure of the liquid, and thus the shape of the tube 30 is likely to be restored.

For this reason, the shape of the tube 30 is further reliably restored between a time when the tube 30 is pressed and deformed and a time when the tube 30 is to be pressed next, and thus a transfer amount of the liquid is stable every time the tube 30 is pressed, thereby improving the flow rate accuracy.

### <Valve Mechanism Unit>

The valve mechanism unit includes the upstream side valve 51 and the upstream side valve cam 51C on the upstream side thereof, and includes the downstream side valve 52 and the downstream side valve cam 52C on the downstream side thereof.

The position of the valve 51 (52) that blocks up the tube 30 and that releases the blocking-up of the tube 30 is determined by the position of the valve cam 51C (52C) that rotates around the shaft 54 as a rotating shaft.

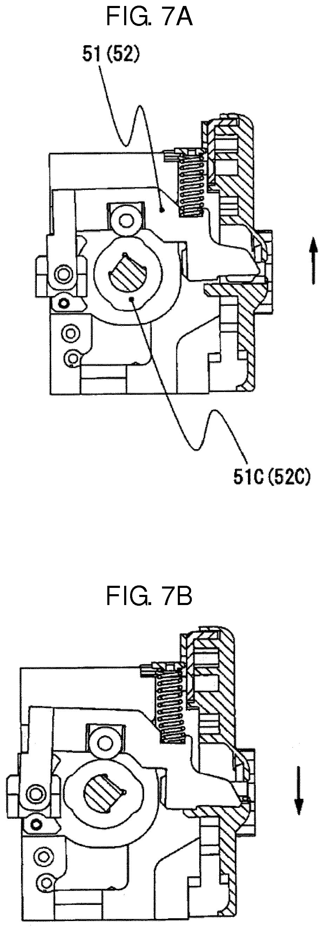

FIG. 7A shows a positional relationship between the valve 51 (52) and the valve cam 51C (52C) when the valve 51 (52) is located at a position at which the valve 51 (52) releases the blocking-up of the tube 30. FIG. 7B shows a positional relationship between the valve 51 (52) and the valve cam 51C (52C) when the valve 51 (52) is located at a position at which the valve 51 (52) blocks up the tube 30.

Since the upstream side valve 51 and the downstream side valve 52 are separate components, an ideal valve angle may be set by largely forming a movable unit, and thus the flow rate accuracy is improved as the infusion pump is improved.

### <Operational Relationship between Pump Block and Valve>

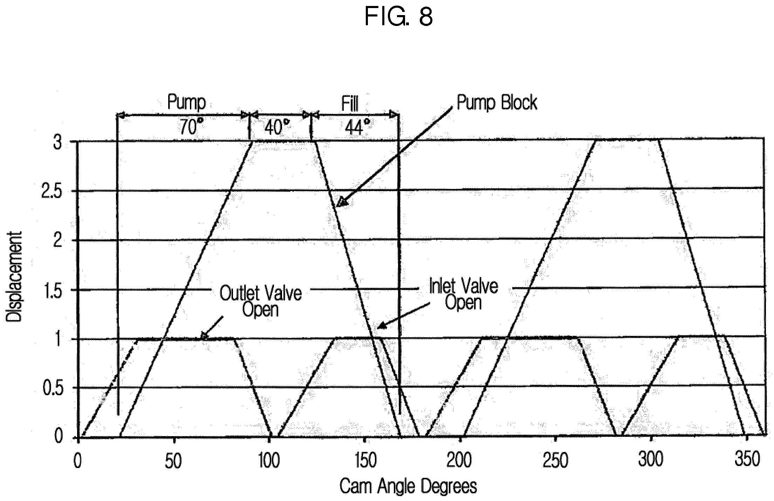

FIG. 8 is a timing chart showing operation timings of the pump block 53, the upstream side valve 51, and the downstream side valve 52 when the shaft 54 rotates one revolution in the tubing pump 1 of the present embodiment.

When the shaft 54 rotates one revolution, the pump block 53 vertically reciprocates once. When the shaft 54 rotates one revolution, the upstream side valve 51 performs an operation for opening and closing the valve twice. At this time, the downstream side valve 52 performs the operation for opening and closing the valve twice at timings when the upstream side valve 51 and downstream side valve 52 alternate with each other. FIG. 8 shows the operational relationship between them.

A horizontal axis of FIG. 8 represents an angle at which three different rotating bodies, that is, the pump block cam 53C, the upstream side valve cam 51C, and the downstream side valve cam 52C, rotate around the shaft 54 as a rotating shaft.

A vertical axis of FIG. 8 represents a movement displacement of the pump block 53, the upstream side valve 51, and the downstream side valve 52.

As shown in FIG. 8, in the present embodiment, the movement displacement is set to 1 when the valve is opened to a maximum and a maximum displacement in which the pump block 53 moves upward or downward from the intermediate position is set to 3.

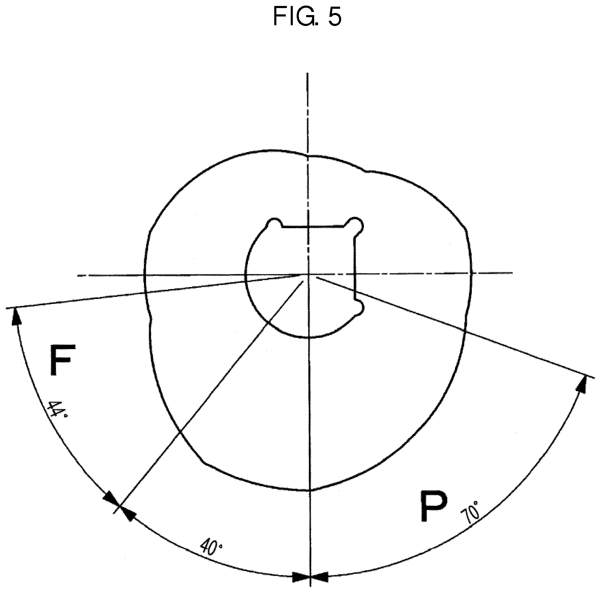

When the liquid in the tube 30 is sucked (filled), the pump block cam 53C brings the pump block 53 into operation in a range of 44° indicated as "F" in FIG. 5. At this time, the upstream side valve 51 opens the infusion tube 30, and the downstream side valve 52 blocks up the infusion tube 30. In addition, as the pump block 53 further moves toward the intermediate position, the liquid is sucked (filled).

When the liquid in the tube 30 is discharged (pumped), the pump block cam 53C brings the pump block 53 into operation in a range of 70° indicated as "P" in FIG. 5. At this time, the upstream side valve 51 blocks up the infusion tube 30, and the downstream side valve 52 opens the infusion tube 30. In addition, the liquid is discharged by causing the inclined surface of the V-groove 53v of the pump block 53 to press the infusion tube 30.

As one of the elements for increasing the flow rate accuracy of the infusion pump, it is preferable to increase the proportion of a discharging time as large as possible after considering various conditions such as the opening and closing timings of the valve or elastic force of the tube.

It is necessary to widely set a cam angle range of the pump block cam 53C for discharging (pumping). In the related art in which the pumping operation is performed using a driving component for pressing a tube and a cam having one upstream side valve and one downstream side valve, it is not likely that the cam angle range is set to be larger than 60°.

However, in the present embodiment, different cams are used to change the operation angles of the pump block 53, the upstream side valve 51, and the downstream side valve 52, and thus a design allowable range is widened. As a result, the cam angle range may be set to at least 70°.

In addition, according to the above-described embodiment, the cam angle range for discharging (pumping) is set to 70°, and the cam angle range for sucking (filling) is set to 44°. However, any of different angle ranges may be set in accordance with the setting of the opening and closing timings of the upstream and downstream side valves.

Further, according to the above-described embodiment, one shaft 54 is used as a common rotating shaft of the pump block cam 53C, the upstream side valve cam 51C, and the downstream side valve cam 52C. However, different rotating shafts may be set with respect to all or some of them. According to the present invention, a tubing pump capable of further improving flow rate accuracy can be configured.

### EXPLANATIONS OF LETTERS OR NUMERALS

1 Tubing pump
10 Pump body
11 Base
20 Door
21 Pump block facing component
21v V-groove
30 Tube
40 Clamping device
50 Pump mechanism
51 Upstream side valve
51C Upstream side valve cam
52 Downstream side valve
52C Downstream side valve cam
53 Pump block
53C pump block cam
53b Ball bushing units
53d Lower bearing unit
53u Upper bearing unit
53v V-groove
53S Guide shaft
54 Shaft

## Claims

1. A tubing pump that transfers a liquid in a tube (30), which is detachably installed, comprises a pump mechanism (50), wherein the pump mechanism (50) comprises a valve mechanism unit that is configured to block up and open the installed tube (30), and a tube pressing mechanism unit that is configured to repeatedly press the tube (30), the tube pressing mechanism unit comprising a pump block (53) that is movably held by at least two guide shafts (53S) extending into the pump block (53) and the tube pressing mechanism being configured so that the tube (30) is repeatedly pressed by a continuous reciprocation of the pump block (53), a reciprocating direction of the pump block (53) being parallel to the extension of the guide shafts (54), wherein the tube pressing mechanism unit is provided with a facing component (21) which does not move when transferring the liquid in the tube (30), the facing component (21) being disposed at a position facing the pump block (53), a first groove portion having an approximately V shape is formed in the pump block (53), a second groove portion having an approximately V shape is formed in the facing component (21), and the first and the second groove portions being configured to accommodate the tube (30) so that the tube (30) is repeatedly pressed between the first groove portion and the second groove portion when the pump block (53) continuously reciprocates.

2. The tubing pump of claim 1, wherein the valve mechanism unit is disposed on an upstream side and a downstream side, the tube pressing mechanism unit is disposed between the valve mechanism unit on the upstream side and the valve mechanism unit on the downstream side, an operation range of the pump block (53) disposed in the tube pressing mechanism unit is determined by a pump block cam (53C), an operation range of an upstream side valve (51) of the valve mechanism unit on the upstream side is determined by a first valve cam (51C), an operation range of a downstream side valve (52) of the valve mechanism unit on the downstream side is determined by a second valve cam (52C), and the pump block cam (53C), the first valve cam (51C), and the second valve cam (52C) are different cam components to each other.

3. The tubing pump according to claim 2, wherein a cam angle range of the pump block cam (53C) for discharging is set to at least 70°.

## Patentansprüche

1. Schlauchpumpe, die eine Flüssigkeit in einem Schlauch (30) überführt, der lösbar angebracht ist, mit einem Pumpenmechanismus (50), wobei der Pumpenmechanismus (50) eine Ventilmechanismuseinheit, die so eingerichtet ist, dass sie den angebrachten Schlauch (30) blockiert bzw. öffnet, und eine Schlauchdrückmechanismuseinheit umfasst, die so eingerichtet ist, dass sie den Schlauch (30) wiederholt drückt, wobei die Schlauchdrückmechanismuseinheit einen Pumpenblock (53) umfasst, der von wenigstens zwei sich in den Pumpenblock (53) erstreckenden Führungswellen (53S) beweglich gehalten ist, und der Schlauchdrückmechanismus so eingerichtet ist, dass der Schlauch (30) durch eine kontinuierliche Hin- und Herbewegung des Pumpenblocks (53) wiederholt gedrückt wird, wobei eine Hin- und Herbewegungsrichtung des Pumpenblocks (53) parallel zur Erstreckung der Führungswellen (54) ist, wobei die Schlauchdrückmechanismuseinheit mit einer gegenüberliegenden Komponente (21) versehen ist, die sich beim Überführen der Flüssigkeit in dem Schlauch (30) nicht bewegt, wobei die gegenüberliegende Komponente (21) in einer dem Pumpenblock (53) zugewandten Position angeordnet ist, wobei ein annähernd V-förmiger erster Nutabschnitt in dem Pumpenblock (53) gebildet ist, ein annähernd V-förmiger zweiter Nutabschnitt in der gegenüberliegenden Komponente (21) gebildet ist und der erste und der zweite Nutabschnitt so eingerichtet sind, dass sie den Schlauch (30) so aufnehmen, dass der Schlauch (30) wiederholt zwischen dem ersten Nutabschnitt und dem zweiten Nutabschnitt gedrückt wird, wenn sich der Pumpenblock (53) kontinuierlich hin- und her bewegt.

2. Schlauchpumpe nach Anspruch 1, bei der die Ventilmechanismuseinheit auf einer stromaufwärtigen Seite und einer stromabwärtigen Seite angeordnet ist, die Schlauchdrückmechanismuseinheit zwischen der Ventilmechanismuseinheit auf der stromaufwärtigen Seite und der Ventilmechanismuseinheit auf der stromabwärtigen Seite angeordnet ist, ein Arbeitsbereich des in der Schlauchdrückmechanismuseinheit angeordneten Pumpenblocks (53) durch eine Pumpenblockkurvenscheibe (53C) bestimmt ist, ein Arbeitsbereich eines stromaufwärtsseitigen Ventils (51) der Ventilmechanismuseinheit auf der stromaufwärtigen Seite durch eine erste Ventilkurvenscheibe (51C) bestimmt ist, ein Arbeitsbereich eines stromabwärtsseitigen Ventils (52) der Ventilmechanismuseinheit auf der stromabwärtigen Seite durch eine zweite Ventilkurvenscheibe (52C) bestimmt ist und die Pumpenblockkurvenscheibe (53C), die erste Ventilkurvenscheibe (51C) und die zweite Ventilkurvenscheibe (52C) voneinander verschiedene Kurvenscheibenkomponenten sind.

3. Schlauchpumpe nach Anspruch 2, bei der ein Kurvenscheibenwinkelbereich der Pumpenblockkurvenscheibe (53C) zum Ablassen auf mindestens 70° eingestellt ist.

## Revendications

1. Pompe tubulaire qui transporte un liquide dans un tube (30) installé de manière détachable, comprenant un mécanisme à pompe (50), le mécanisme à pompe (50) comprenant une unité de mécanisme à valve réalisée de manière à bloquer et ouvrir le tube installé (30), et une unité de mécanisme de compression de tube qui est réalisée de manière à comprimer le tube (30) de façon répétée, l'unité de mécanisme de compression de tube comprenant un bloc de pompage (53) qui est retenu de manière mobile par au moins deux arbres de guidage (53S) qui s'étendent dans le bloc de pompage (53), et le mécanisme de compression de de tube étant réalisé de telle sorte que le tube (30) est comprimé de façon répétée par un mouvement alternatif continu du bloc de pompage (53), un sens de mouvement alternatif du bloc de pompage (53) étant parallèle à l'extension des arbres de guidage (54),
dans laquelle l'unité de mécanisme de compression de tube est pourvue d'un composant de face (21) qui ne se déplace pas lors du transport du liquide dans le tube (30), le composant de face (21) étant agencé dans une position en face du bloc de pompage (53), un premier tronçon de rainure présentant une forme approximativement en V est réalisé dans le bloc de pompage (53), un deuxième tronçon de rainure présentant une forme approximativement en V est réalisé dans le composant de face (21), et le premier et le deuxième tronçon de rainure étant réalisés pour loger le tube (30) de sorte que le tube (30) est comprimé de manière répétée entre le premier tronçon de rainure et le deuxième tronçon de rainure lorsque le bloc de pompage (53) effectue continuellement un mouvement alternatif.

2. Pompe tubulaire selon la revendication 1, dans laquelle l'unité de mécanisme à valve est agencée d'un côté amont et d'un côté aval, l'unité de mécanisme de compression de tube est agencée entre l'unité de mécanisme à valve du côté amont et l'unité de mécanisme à valve du côté aval, une plage de fonctionnement du bloc de pompage (53) agencé dans l'unité de mécanisme de compression de tube est déterminée par une came (53C) de bloc de pompage, une plage de fonctionnement d'une valve (51) côté amont de l'unité de mécanisme à valve du côté amont est déterminée par une première came de valve (51C), une plage de fonctionnement d'une valve (52) côté aval de l'unité de mécanisme à valve du côté aval est déterminée par une deuxième came de valve (52C), et la came (53C) de bloc de pompage, la première came de valve (51C), et la deuxième came de valve (52C) sont des composants de came différents les uns des autres.

3. Pompe tubulaire selon la revendication 2, dans laquelle une plage angulaire de came de la came (53C) de bloc de pompage pour la décharge est fixée à au moins 70°.
